(19) Europäisches Patentamt
European Patent Office
Office européen des brevets

(11) **EP 4 076 731 B1**

(12) **FASCICULE DE BREVET EUROPEEN**

(45) Date de publication et mention
de la délivrance du brevet:
**11.12.2024  Bulletin 2024/50**

(21) Numéro de dépôt: **20848847.8**

(22) Date de dépôt: **18.12.2020**

(51) Classification Internationale des Brevets (IPC):
*B01J 20/18* (2006.01)    *B01J 20/28* (2006.01)
*B01J 20/30* (2006.01)

(52) Classification Coopérative des Brevets (CPC):
**B01J 20/18; B01J 20/28011; B01J 20/2803;
B01J 20/28042; B01J 20/3028; B01J 20/3078;
B01J 20/3085**

(86) Numéro de dépôt international:
**PCT/FR2020/052530**

(87) Numéro de publication internationale:
**WO 2021/123662 (24.06.2021 Gazette 2021/25)**

(54) **ADSORBANT ZÉOLITHIQUE POUR LA SÉPARATION D'ISOMÈRES D'HYDROCARBURES**

ZEOLITHADSORBENS ZUR TRENNUNG VON KOHLENWASSERSTOFFISOMEREN

ZEOLITE ADSORBENT FOR SEPARATING HYDROCARBON ISOMERS

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité:  **20.12.2019  FR 1915341**

(43) Date de publication de la demande:
**26.10.2022  Bulletin 2022/43**

(73) Titulaires:
• **IFP Energies nouvelles
92500 Rueil-Malmaison (FR)**
• **ARKEMA FRANCE
92700 Colombes (FR)**

(72) Inventeurs:
• **PEREZ-PELLITERO, Javier
92852 RUEIL-MALMAISON Cedex (FR)**
• **BOUVIER, Ludivine
64170 LACQ (FR)**
• **MANKO, Maria
92852 RUEIL-MALMAISON Cedex (FR)**
• **MOREAUD, Maxime
92852 RUEIL-MALMAISON Cedex (FR)**

(74) Mandataire: **Bandpay & Greuter
11 rue Christophe Colomb
75008 Paris (FR)**

(56) Documents cités:
**WO-A1-2018/002174    DE-A1- 102018 109 701**

**Description**

DOMAINE DE L'INVENTION

L'invention concerne le domaine des adsorbants zéolithiques sous forme d'agglomérés comprenant de la zéolithe de type faujasite, pour la séparation de mélanges gazeux ou liquides d'hydrocarbures aromatiques, et concerne plus particulièrement les procédés de séparation des xylènes et notamment des procédés de séparation de *para*-xylène à productivité améliorée.

La présente invention concerne également un procédé de séparation de mélanges gazeux ou liquides d'isomères à productivité améliorée, et plus particulièrement un procédé de séparation des isomères du xylène à productivité améliorée pour la production de *para*-xylène très pur à partir d'une charge d'hydrocarbures aromatiques contenant des isomères à 8 atomes de carbone.

TECHNIQUE ANTERIEURE

L'utilisation d'adsorbants zéolithiques constitués de zéolithes Faujasite (FAU) de type X ou Y comprenant, outre des cations sodium, des ions baryum, potassium ou strontium, seuls ou en mélanges, pour adsorber sélectivement le *para*-xylène dans un mélange d'hydrocarbures aromatiques, est bien connue de l'art antérieur.

Les brevets US3558730, US3558732, US3626020 et US3663638 montrent que des adsorbants zéolithiques comprenant des aluminosilicates à base de sodium et de baryum (US3960774) ou à base de sodium, de baryum et de potassium, sont efficaces pour la séparation du *para*-xylène présent dans des coupes aromatiques en C8 (coupes comprenant des hydrocarbures aromatiques à 8 atomes de carbone).

Les adsorbants décrits dans le brevet US3878127 sont utilisés comme agents d'adsorption dans les procédés en phase liquide, de préférence de type contre-courant simulé, similaires à ceux décrits dans le brevet US2985589 et qui s'appliquent, entre autres, aux coupes aromatiques en C8.

L'invention a pour but d'améliorer la productivité des procédés de préparation de *para*-xylène existants et notamment les procédés en phase liquide, de préférence de type contre-courant simulé de séparation des isomères de xylène à partir de charges aromatiques en C8. Il a été observé de manière surprenante que cette productivité peut être améliorée par le choix judicieux des caractéristiques des agglomérés adsorbants zéolithiques mis en oeuvre dans ce type de procédés.

La séparation en lit mobile simulé s'entend ici au sens large, c'est-à-dire qu'on peut avoir affaire soit à un lit mobile à contre-courant simulé, soit à un lit mobile à co-courant simulé, soit à un procédé dit "Varicol". Le procédé Varicol proposé par Ludemann-Hombourger (O. Ludemann-Hombourger, R. Nicoud, 2000) et développé plus tard par l'entreprise Novasep, permet un déplacement non synchronisé des points d'injection et de soutirage (Bailly, *et al.* 2004). Les longueurs des quatre zones peuvent être ajustées pendant le cycle en limitant le nombre de lits nécessaire pour réaliser la séparation.

La caractéristique commune à cette famille de procédés est que l'aggloméré adsorbant zéolithique (ou plus simplement « solide adsorbant ») est mis en oeuvre en lit fixe, et que les flux liquides en contact avec le solide adsorbant sont gérés soit au moyen d'un jeu de vannes « tout ou rien », soit au moyen d'une vanne unique complexe connue sous le nom de vanne rotative ou "rotary valve".

Lorsque l'élément actif des solides adsorbants utilisés comme agents d'adsorption dans ces procédés est une zéolithe, cette dernière, obtenue sous forme de cristaux, est de préférence employée à l'échelle industrielle sous forme d'agglomérés. Ces adsorbants zéolithiques, agglomérés sous forme de plaquettes, billes ou extrudés, sont en général constitués de cristaux de zéolithe, qui constitue l'élément actif au sens de l'adsorption et d'un liant destiné à assurer la cohésion des cristaux sous forme d'agglomérés. Ce liant confère également aux agglomérés une résistance mécanique suffisante pour résister aux contraintes mécaniques auxquels ils sont soumis au cours de leur mise en oeuvre au sein des unités. Ces contraintes mécaniques sont à l'origine de la formation de « fines », qui induisent une détérioration des performances au cours de la durée d'opération du procédé.

Le procédé de séparation des xylènes en lit mobile simulé (LMS) a connu de très nombreuses améliorations technologiques, notamment au niveau des plateaux distributeurs des fluides, mais relativement peu d'évolution concernant les caractéristiques intrinsèques du solide adsorbant.

Les adsorbants pour la séparation des xylènes présentent des propriétés de transfert améliorées pour la séparation des xylènes sont par exemple décrits dans la demande internationale WO2008009845 qui décrit des adsorbants à base de zéolithe X, à petits cristaux, de taille inférieure à 1,7 $\mu$m, de rapport atomique Si/Al, tel que 1,15 < Si/Al ≤ 1,5, échangés au baryum, et éventuellement au potassium, dans la demande internationale WO2014090771, qui décrit des adsorbants zéolithiques agglomérés, à propriétés optimisées, notamment pour la séparation du *para*-xylène des coupes aromatiques en C8. Ces adsorbants présentent des propriétés maximales de sélectivité vis-à-vis du *para*-xylène et de transfert de matière, tout en présentant une résistance mécanique maximale associée à une capacité d'adsorption optimisée.

La demande internationale WO2018002174 propose des adsorbants zéolithiques sous forme d'agglomérés aux propriétés optimisées pour la séparation de mélanges gazeux ou liquides d'isomères et plus particulièrement pour la séparation des xylènes, en phase gaz ou en phase liquide, notamment du *para*-xylène des coupes aromatiques en C8. Les adsorbants zéolithiques de l'invention présentent notamment des propriétés maximales de sélectivité vis-à-vis du *para*-xylène et de transfert de matière, tout en présentant une résistance améliorée et une capacité d'adsorption par volume d'adsorbant élevées et sont particulièrement adaptés pour une utilisation dans un procédé de séparation du *para*-xylène en phase liquide, de préférence de type contre-courant simulé.

En particulier, cette demande enseigne qu'il n'est pas souhaitable d'augmenter fortement la macroporosité et/ou mésoporosité, donc la porosité de grain car cette porosité ne participe pas à la capacité d'adsorption. L'optimisation des propriétés de diffusion et des capacités d'adsorption optimales, ont été obtenues en sélectionnant de manière spécifique à la fois la porosité et le facteur de tortuosité.

La demande de brevet US2009/0326308 décrit un procédé de séparation au moyen d'un adsorbant à faible teneur en liant et à base de cristaux de faujasite de type X de taille nanométrique, typiquement de taille moyenne inférieure à 500 nm. La demande de brevet DE 10 2018 109701 A1 divulgue un adsorbant zéolithique aggloméré, comprenant au moins une zéolithe faujasite de rapport atomique Si/ Al compris entre 1,00 et 1,50, caractérisé en ce que la porosité de grain dudit adsorbant est de préférence au moins 35%; ledit adsorbant comprend des cristaux de zéolithe de diamètre compris entre 0,1 $\mu$m et 20 $\mu$m, et se présente sous la forme de billes.

Dans le procédé de séparation des xylènes par adsorption en lit mobile simulé, l'adsorbant zéolithique est mis en contact avec le flux liquide d'alimentation (charge) comprenant le plus souvent des mélanges d'hydrocarbures en C8, et généralement et le plus souvent des mélanges d'isomères du xylène et plus particulièrement des mélanges de *ortho*-xylène, *méta*-xylène, *para*-xylène et éthylbenzène.

En utilisant un adsorbant zéolithique à base de zéolithe de structure faujasite de rapport Si/Al compris entre 1,0 et 1,5 (zéolithes LSX, MSX, X) échangée au baryum ou échangée très majoritairement au baryum et de manière minoritaire au potassium, le *para*-xylène est alors adsorbé dans les micropores de la zéolithe préférentiellement par rapport à l'ensemble des autres composés hydrocarbures présents dans le flux d'alimentation. La phase adsorbée dans les micropores de la zéolithe se trouve alors enrichie en *para*-xylène par rapport au mélange initial constituant le flux d'alimentation. La phase liquide se trouve à l'inverse enrichie en composés tels que, *ortho*-xylène, *méta*-xylène et éthylbenzène en proportion relative plus importante que celle caractérisant le mélange initial constituant le flux d'alimentation.

La phase liquide est soustraite du contact avec l'adsorbant formant ainsi un flux de raffinat. La phase adsorbée, enrichie en *para*-xylène, est désorbée sous l'action d'un flux de désorbant, et soustraite du contact avec l'adsorbant formant alors un flux d'extrait.

Dans le procédé de séparation des xylènes par adsorption en lit mobile simulé, le solide adsorbant zéolithique est mis en oeuvre dans une ou deux colonnes multi-étagées pour être mis en contact avec le flux de liquide. On appelle colonne multi-étagée une colonne constituée d'une multiplicité de plateaux disposés selon un axe sensiblement vertical, chaque plateau supportant un lit de solide granulaire, et les différents lits successifs étant traversés en série par le ou les fluides mis en oeuvre dans la colonne. Entre deux lits successifs est situé un dispositif de distribution des fluides permettant d'alimenter chaque lit de solide granulaire.

De manière générale le fonctionnement d'une colonne en lit mobile simulé peut être décrit de la façon suivante: Une colonne comporte au moins quatre zones, et éventuellement cinq ou six, chacune de ces zones étant constituée par un certain nombre de lits successifs, et chaque zone étant définie par sa position comprise entre un point d'alimentation et un point de soutirage. Typiquement, une unité CCS pour la production de *para*-xylène est alimentée par au moins une charge F à fractionner (charge d'hydrocarbures aromatiques constituée des isomères à 8 atomes de carbones) et un désorbant D, parfois appelé éluant (généralement du *para*-diéthylbenzène ou du toluène), et l'on soutire de ladite unité au moins un raffinat R contenant les produits de la charge les moins sélectivement adsorbés et du désorbant et un extrait E contenant le produit de la charge le plus adsorbé et du désorbant.

D'autres points d'injection et de soutirage peuvent être ajoutés de manière à rincer les circuits de distribution, comme décrit par exemple dans le brevet US7208651. L'ajout de ces flux supplémentaires de rinçage ne changeant en rien le principe de fonctionnement du CCS, dans un souci de concision, nous n'ajouterons pas ces points d'injection et de soutirage supplémentaires dans la description du procédé selon l'invention.

Les points d'alimentation et de soutirage sont modifiés au cours du temps, décalés dans le même sens d'une valeur correspondant à un lit. Les décalages des différents points d'injection ou de soutirage peuvent être soit simultanés, soit non-simultanés comme l'enseigne le brevet US6136198. Le procédé selon ce second mode de fonctionnement est appelé Varicol.

Classiquement, on définit 4 zones chromatographiques différentes dans une colonne fonctionnant en contre-courant simulé (CCS).

- Zone 1 : zone de désorption du produit de la charge le plus adsorbé, comprise entre l'injection du désorbant D et le prélèvement de l'extrait E.
- Zone 2 : zone de désorption des produits de la charge les moins sélectivement adsorbés, comprise entre le prélèvement de l'extrait E et l'injection de la charge à fractionner F.
- Zone 3 : zone d'adsorption du produit de la charge le plus adsorbé, comprise entre l'injection de la charge et le soutirage du raffinat R.
- Zone 4 : zone située entre le soutirage de raffinat R et l'injection du désorbant D.

Pour augmenter la productivité du procédé de séparation, l'art antérieur enseigne qu'une manière consiste à améliorer le transfert global dans l'aggloméré adsorbant zéolithique et notamment en diminuant la taille des cristaux et/ou la taille moyenne desdits agglomérés.

Les documents de l'art antérieur décrivant des procédés de séparation pharmaceutique (Gomes et al., (2006), Adsorption, vol. 12, p. 375 sqq.) rapportent des procédés de séparation chromatographique en phase liquide utilisant des agglomérés de quelques dizaines de micromètres à 100 $\mu$m.

Dans ces procédés utilisant des adsorbants de très petite taille, la perte de charge $\Delta P$ est alors élevée. Dans les procédés de séparation des xylènes, de tels niveaux de perte de charge $\Delta P$ ne sont pas courants. On remarque néanmoins de façon étonnante que la perte de charge $\Delta P$ n'est pas un critère dimensionnant. Il aura un impact notamment sur l'épaisseur des parois de l'adsorbeur et sur la puissance des installations.

Une autre caractéristique de l'aggloméré adsorbant zéolithique est le taux d'hydratation dudit aggloméré. Le maintien de l'hydratation de la zéolithe à la valeur souhaitée, par exemple une perte au feu de 4% à 7,7% pour la zéolithe X, MSX ou LSX, lors de sa mise en oeuvre dans le procédé de séparation des xylènes par adsorption en lit mobile simulé, est assuré en ajoutant de l'eau dans les flux d'alimentation de la charge et/ou de désorbant. L'addition d'eau nécessaire pour de tels niveaux de perte au feu est telle que la teneur en eau en poids dans les effluents hydrocarbonés (le flux d'extrait ou de raffinat) est le plus souvent comprise entre 0 ppm et 150 ppm, et plus généralement entre 40 ppm et 150 ppm, lorsque l'adsorbant est à base de zéolithe X, MSX ou LSX.

Néanmoins il est toujours nécessaire d'augmenter la productivité du procédé de manière générale.

La présente invention a ainsi pour premier objet de proposer un adsorbant zéolitique sous forme d'agglomérés aux propriétés optimisées pour la séparation de mélanges gazeux ou liquides d'isomères et plus particulièrement pour la séparation des xylènes, en phase gaz ou en phase liquide, notamment pour la séparation du *para*-xylène à partir de coupes aromatiques en C8. Les agglomérés adsorbants zéolitiques de l'invention présentent notamment des propriétés maximales de sélectivité vis-à-vis du *para*-xylène et de transfert de matière, tout en présentant une résistance mécanique et une capacité d'adsorption élevées et sont particulièrement adaptés pour une utilisation dans un procédé de séparation du *para*-xylène en phase liquide, de préférence de type contre-courant simulé.

La présente invention propose à cet effet un adsorbant aggloméré, préférentiellement à base de zéolithe faujasite de rapport atomique Si/Al compris entre 1,00 et 1,50, et dont la porosité de grain est avantageusement comprise entre 25% et 45%, permettant la production de *para*-xylène de haute pureté avec une productivité améliorée, tout en évitant la dégradation des performances au cours du temps. Plus précisément, l'invention concerne un adsorbant aggloméré zéolithique, comprenant au moins une zéolithe faujasite de rapport atomique Si/Al compris entre 1,00 et 1,50 bornes incluses (FAU-X) et comprenant de préférence du baryum et éventuellement du potassium, dans lequel d'une part la porosité de grain est comprise entre 25% et 45 %, de préférence entre 30% et 45 %, de préférence encore entre 32% et 45% et de manière très préférée entre 35% et 45%, de manière particulièrement avantageuse entre 36% et 45%, bornes incluses, et d'autre part en ce que la déviation standard $\sigma$ de la distribution de taille de cristaux dans ledit aggloméré est inférieure à 0,30 $\mu$m, de préférence comprise entre 0,05 $\mu$m et 0,30 $\mu$m, de préférence encore entre 0,05 $\mu$m et 0,28 $\mu$m, et de manière tout à fait préférée entre 0,1 $\mu$m et 0,28 $\mu$m, de préférence entre toutes entre 0,1 $\mu$m et 0,25 $\mu$m, bornes incluses.

RESUME DE L'INVENTION

Ainsi, et selon un premier aspect, l'invention concerne un adsorbant zéolithique aggloméré, comprenant au moins une zéolithe faujasite de rapport atomique Si/Al compris entre 1,00 et 1,50 bornes incluses (FAU-X) et comprenant du baryum et éventuellement du potassium, caractérisé d'une part en ce que la porosité de grain dudit adsorbant est comprise entre 25% et 45 %, de préférence entre 30% et 45 %, de préférence encore entre 32% et 45% et de manière très préférée entre 35% et 45%, de manière particulièrement avantageuse entre 36% et 45%, bornes incluses, et d'autre part en ce que la déviation standard $\sigma$ de la distribution de taille de cristaux dans ledit aggloméré est inférieure à 0,30 $\mu$m, de préférence comprise entre 0,05 $\mu$m et 0,30 $\mu$m, de préférence encore entre 0,05 $\mu$m et 0,28 $\mu$m, et de manière tout à fait préférée entre 0,1 $\mu$m et 0,28 $\mu$m, de préférence entre toutes entre 0,1 $\mu$m et 0,25 $\mu$m, bornes incluses, ledit adsorbant comprenant des cristaux de zéolithe de diamètre moyen en nombre compris entre 100 nm et 1200 nm et la fraction massique de zéolithe FAU étant supérieure ou égale à 80% par rapport au poids total d'adsorbant.

Selon un mode de réalisation, l'adsorbant zéolithique aggloméré selon l'invention comprend des cristaux de zéolithe de diamètre moyen en nombre compris entre 400 nm et 1200 nm, plus préférentiellement entre 500 nm et 1200 nm, de manière tout à fait préférée entre 550 nm et 1200 nm, très avantageusement entre 600 nm et 1200 nm, bornes incluses.

Selon un autre mode de réalisation, l'adsorbant zéolithique aggloméré selon l'invention se présente sous la forme de billes de diamètre moyen compris entre 100 $\mu$m et 1000 $\mu$m, de préférence entre 100 $\mu$m et 600 $\mu$m, de préférence encore 200 $\mu$m et 550 $\mu$m, bornes incluses.

Dans un mode de réalisation préféré, ladite au moins une zéolithe FAU-X de l'adsorbant zéolithique aggloméré selon l'invention présente un rapport atomique Si/Al compris entre 1,05 et 1,50, de préférence entre 1,05 et 1,40, bornes incluses et de manière encore plus préférée entre 1,10 et 1,40 bornes incluses.

Dans un autre mode de réalisation préféré de l'invention, aucune structure zéolithique autre que la structure faujasite, de préférence aucune structure zéolithique autre que la structure faujasite X, n'est détectée par diffraction des rayons X, dans l'adsorbant zéolithique aggloméré selon l'invention.

L'adsorbant zéolithique aggloméré selon l'invention comprend un ou plusieurs ion(s) alcalin(s) ou alcalino-terreux, de préférence choisi(s) parmi sodium, baryum et potassium.

Selon un mode de réalisation préféré, la teneur en baryum, exprimée en oxyde de baryum (BaO) dans l'adsorbant zéolithique aggloméré selon l'invention est supérieure à 10%, de préférence encore supérieure à 15%, de manière très préférée supérieure à 20%, de manière encore plus préférée supérieure à 23 %, voire supérieure à 33% en poids par rapport à la masse totale de l'adsorbant, et avantageusement, ladite teneur en baryum est comprise entre 23% et 42%, et typiquement entre 30% et 40%, bornes incluses, en poids par rapport au poids total de l'adsorbant.

Selon un autre mode de réalisation préféré, la teneur en potassium, exprimée en oxyde de potassium ($K_2O$) dans l'adsorbant zéolithique aggloméré selon l'invention est inférieure à 25%, de préférence comprise entre 0 et 20%, de manière encore plus préférée comprise entre 0 et 15%, bornes incluses, en poids par rapport à la masse totale de l'adsorbant.

Dans un mode de réalisation de la présente invention, la perte au feu de l'adsorbant zéolithique aggloméré selon l'invention, mesurée à 900°C selon la norme NF EN 196-2, est inférieure ou égale à 7,7%, de préférence comprise entre 0 et 7,7%, de manière préférée entre 3,0% et 7,7%, de manière encore préférée entre 3,5% et 6,5% et avantageusement entre 4,5% et 6%, bornes incluses.

Selon un autre aspect, la présente invention concerne l'utilisation de l'adsorbant zéolithique aggloméré tel qu'il vient d'être décrit dans les procédés de :

- séparation de coupes d'isomères aromatiques en C8 et notamment des xylènes, et plus particulièrement de *para*-xylène,
- séparation d'isomères de toluène substitué tels que nitrotoluène, diéthyltoluène, toluènediamine, et autres,
- séparation des crésols,
- séparation des alcools polyhydriques.

Enfin, et selon encore un autre aspect, la présente invention concerne le procédé de séparation de *para*-xylène à partir de coupes d'isomères aromatiques à 8 atomes de carbone, utilisant comme agent d'adsorption du *para*-xylène, un adsorbant zéolithique aggloméré tel qu'il a été défini précédemment et plus précisément dans la suite du présent exposé.

Le procédé de séparation de *para*-xylène à partir de coupes d'isomères d'hydrocarbures aromatiques contenant 8 atomes de carbone, de la présente invention, est conduit en phase gazeuse ou en phase liquide, de préférence en phase liquide, par adsorption du *para*-xylène en présence d'un désorbant, ledit désorbant étant de préférence choisi parmi le toluène et le *para*-diéthylbenzène.

Selon un mode de réalisation préféré, le procédé de séparation de *para*-xylène selon la présente invention, est un procédé de type lit mobile simulé, de préférence encore de type à contre-courant simulé.

DESCRIPTION DES MODES DE REALISATION

L'adsorbant zéolithique de l'invention comprend de préférence à la fois des macropores, des mésopores et des micropores. Par « macropores », on entend des pores dont l'ouverture est supérieure à 50 nm, de préférence comprise entre 50 nm et 400 nm. Par « mésopores », on entend des pores dont l'ouverture est comprise entre 2 nm et 50 nm, bornes non incluses. Par « micropores », on entend des pores dont l'ouverture est inférieure à 2 nm.

Comme indiqué précédemment, l'adsorbant selon la présente invention se présente sous forme d'un adsorbant dont la porosité de grain est comprise entre 25% et 45 %, de préférence entre 30% et 45 %, de préférence encore entre 32% et 45%, de manière très préférée entre 35% et 45%, et de manière particulièrement avantageuse entre 36% et 45%bornes incluses. En outre, la déviation standard $\sigma$ de la distribution de taille de cristaux dans ledit adsorbant est inférieure à 0,30 μm, de préférence comprise entre 0,05 μm et 0,30 μm, de préférence encore entre 0,05 μm et 0,28 μm, et de manière tout à fait préférée entre 0,10 μm et 0,28 μm, de préférence entre toutes entre 0,10 μm et 0,25 μm, bornes incluses. Ledit adsorbant comprend des cristaux de zéolithe de diamètre moyen en nombre compris entre 100 nm et 1200 nm, et la fraction massique de zéolithe FAU est supérieure ou égale à 80% par rapport au poids total d'adsorbant

En effet, les inventeurs ont découvert de manière surprenante que lorsque la déviation standard σ de la distribution de taille de cristaux dans l'adsorbant zéolithique est supérieure à 0,30 μm, on observe une chute drastique de la productivité dans un procédé de séparation de *para*-xylène, notamment dans un procédé de séparation en phase liquide, à contre-courant en lit mobile simulé.

Il a également été observé de manière surprenante que cette valeur de productivité atteint un maximum pour des valeurs de déviation standard σ de la distribution de taille de cristaux dans l'aggloméré adsorbant zéolithique inférieure à 0,30 μm. Cette valeur de déviation standard σ paraît correspondre à une porosité de grain optimisée. En effet, il a été également observé de façon tout à fait surprenante que la porosité de grain est inversement proportionnelle à la déviation standard σ de la distribution de taille de cristaux dans l'aggloméré adsorbant zéolithique. Ainsi, plus la déviation standard σ diminue, plus la porosité de grain augmente. Or une porosité de grain trop importante conduit à des effets tout à fait non désirés, tels que par exemple perte de capacité d'adsorption, perte en résistance mécanique, et autres.

Par conséquent, l'homme du métier souhaitant une productivité maximale trouve grâce à la présente invention un compromis entre la porosité du grain et la déviation standard σ de la distribution de taille de cristaux dans l'aggloméré adsorbant zéolithique. Grâce à l'adsorbant aggloméré selon l'invention, il est ainsi possible d'obtenir une productivité maximale dans les procédés de séparation des xylènes.

Avantageusement, l'adsorbant zéolithique aggloméré se présente sous la forme de billes de diamètre moyen compris entre 100 μm et 1000 μm, de préférence entre 100 μm et 600 μm, de préférence encore 200 μm et 550 μm, bornes incluses.

De préférence, l'adsorbant zéolithique à base de faujasite selon l'invention comprend du baryum et éventuellement du potassium.

Selon encore un autre mode de réalisation de l'invention, l'adsorbant zéolithique présente une teneur en baryum, exprimée en oxyde de baryum (BaO) supérieure à 10%, de préférence supérieure à 15%, de manière très préférée supérieure à 20%, de manière encore plus préférée supérieure à 23%, voire supérieure à 33% en poids par rapport à la masse totale de l'adsorbant. Avantageusement, la teneur en baryum est comprise entre 23% et 42%, et typiquement entre 30% et 40%, bornes incluses, en poids par rapport au poids total de l'adsorbant.

Selon un autre mode réalisation de l'invention, l'adsorbant zéolithique peut présenter une teneur en potassium, exprimée en oxyde de potassium $K_2O$ inférieure à 25%, de préférence comprise entre 0 et 20%, de manière encore plus préférée comprise entre 0 et 15%, bornes incluses, en poids par rapport à la masse totale de l'adsorbant.

Selon un autre mode de réalisation de l'invention la teneur totale en ions alcalins ou alcalino-terreux autres que le baryum et le potassium, exprimée en oxydes d'ions alcalins ou alcalino-terreux autres que l'oxyde de baryum BaO et l'oxyde de potassium $K_2O$ est comprise entre 0 et 5%, bornes incluses, par rapport à la masse totale de l'adsorbant.

De préférence l'adsorbant zéolithique selon la présente invention est un adsorbant à base de zéolithe(s) FAU, généralement référencée(s) sous le nom de zéolithe de type X. Par « zéolithe X », on entend une zéolithe dont le rapport atomique Si/Al est compris entre 1,00 et 1,50 bornes incluses, de préférence entre 1,00 et 1,40, bornes incluses.

Parmi les zéolithes X, il est maintenant communément admis de reconnaître deux sous-groupes dénommés zéolithes LSX et zéolithes MSX. Les zéolithes LSX présentent un rapport atomique Si/Al égal à environ 1, c'est-à-dire 1,00 ± 0,05, et les zéolithes MSX présentent un rapport atomique Si/Al compris entre environ 1,05 et environ 1,15, bornes exclues.

Selon un mode de réalisation préféré de la présente invention, la zéolithe X présente un rapport atomique Si/Al compris entre 1,15 et 1,50, bornes incluses. Selon un autre mode de réalisation préféré, la zéolithe X est une zéolithe de type LSX de rapport atomique Si/Al est égal à environ 1, c'est-à-dire 1,00 ± 0,05. Il peut également être envisageable que l'adsorbant contienne des mélanges de deux ou plusieurs types de zéolithes X tels qu'ils viennent d'être définis.

Selon un mode de réalisation préféré, ladite au moins une zéolithe FAU comprise dans l'aggloméré adsorbant zéolithique de l'invention présente un rapport atomique Si/Al compris entre 1,00 et 1,50 bornes incluses, de préférence entre 1,05 et 1,40 et de manière encore plus préférée compris entre 1,10 et 1,40. De préférence, ladite au moins une zéolithe FAU est une zéolithe X.

Selon un autre mode de réalisation préféré, aucune structure zéolithique autre que la structure FAU, de préférence aucune structure zéolithique autre que la structure faujasite X, n'est détectée par diffraction des rayons X (connue de l'homme du métier sous l'acronyme DRX) dans l'aggloméré adsorbant zéolithique de la présente invention.

La fraction massique de zéolithe FAU, la zéolithe FAU étant de préférence une zéolithe X, est supérieure ou égale à 80% par rapport au poids total d'adsorbant de la présente invention, le complément à 100% étant de préférence constitué de phase non zéolithique.

Les agglomérés adsorbants zéolithiques selon la présente invention peuvent contenir une phase non zéolithique (PNZ), c'est-à-dire une phase non cristalline qui est essentiellement inerte vis-à-vis de l'adsorption. Le taux de cristallinité (fraction massique de zéolithe) de l'adsorbant selon l'invention peut être mesuré par analyse par diffraction de rayons X, connue de l'homme du métier sous l'acronyme DRX.

L'aggloméré adsorbant zéolithique de l'invention est sous la forme d'un aggloméré, c'est-à-dire qu'il est constitué d'éléments cristallins (ou cristaux) d'au moins une zéolithe FAU telle que définie précédemment, lesdits éléments cristallins (ou plus simplement « cristaux ») présentant un diamètre moyen en nombre compris entre 100 nm et 1200 nm, de préférence encore compris entre 400 nm et 1200 nm, plus préférentiellement entre 500 nm et 1200 nm, de manière tout à fait préférée entre 550 nm et 1200 nm, très avantageusement entre 600 nm et 1200 nm, bornes incluses.

MODES DE REALISATION

Les agglomérés adsorbants zéolithiques selon la présente invention peuvent être préparés en adaptant les modes opératoires déjà connus de l'homme du métier, et comme décrit par exemple dans les documents WO2014090771, WO2018002174, US2009/0326308 précédemment cités et en sélectionnant et ajustant les paramètres de synthèse permettant d'obtenir les agglomérés présentant les valeurs souhaitées de porosité de grain et de déviation standard σ.

Un procédé de synthèse de l'aggloméré adsorbant zéolithique selon la présente invention peut par exemple comprendre au moins les étapes de :

a) agglomération de cristaux d'au moins une zéolithe de type FAU-X, avec un liant comprenant au moins 80% d'argile ou d'un mélange d'argiles zéolithisable(s), et avec éventuellement jusqu'à 5% d'additifs ainsi qu'avec la quantité d'eau qui permet la mise en forme du matériau aggloméré ; séchage des agglomérés à une température comprise entre 50°C et 150°C ; calcination des agglomérés séchés sous balayage gazeux oxydant et/ou inerte, avec notamment des gaz tels que l'oxygène, l'azote, l'air, un air sec et/ou décarbonaté, un air appauvri en oxygène, éventuellement sec et/ou décarbonaté, à une température supérieure à 150°C, typiquement comprise entre 180°C et 800°C, préférentiellement entre 200°C et 650°C,
b) zéolithisation de tout ou partie du liant par mise en contact des agglomérés obtenus à l'étape a) avec une solution basique alcaline,
c) échange(s) cationique(s) des agglomérés de l'étape a) et/ou de l'étape b) par mise en contact avec une solution d'ions baryum et/ou d'ions potassium,
d) échange cationique éventuel supplémentaire des agglomérés de l'étape c) par mise en contact avec une solution d'ions potassium,
e) lavage et séchage des agglomérés obtenus aux étapes c) ou d), à une température comprise entre 50°C et 150°C, et
f) activation par chauffage à une température en général comprise entre 100°C et 400°C, de préférence entre 200°C et 300°C puis récupération de l'adsorbant aggloméré zéolithique.

Les cristaux de zéolithes utilisables dans l'étape a) de synthèse ci-dessus peuvent avantageusement être synthétisés selon les modes opératoires connus et disponibles dans la littérature scientifique ou la littérature brevets, ainsi que sur l'internet. On peut en particulier préparer les cristaux de zéolithes comme décrit dans les documents CN1191118C, ou encore WO2014090771, ou encore US7812208 B2, US2009326308 et US2007224113.

Les paramètres qui peuvent permettre de contrôler la déviation standard σ dans l'aggloméré adsorbant zéolithique de la présente invention sont par exemple la nature même des cristaux utilisés à l'étape a), notamment la taille et la déviation standard desdits cristaux, mais aussi les conditions de zéolithisation du liant d'agglomération, comme par exemple la température, la durée, le pH de la solution alcaline de zéolithisation, ou encore la durée, le mode d'agitation, le taux de cisaillement, pression, et autres.

Plus spécifiquement par « nature des cristaux utilisés à l'étape a) », on entend notamment la déviation standard desdits cristaux qui peut par exemple être contrôlée en ajustant les paramètres de synthèse, et en particulier la température de synthèse, la vitesse d'agitation, le taux de cisaillement, comme indiqué par exemple dans les documents WO2009081022 ou encore US2009326308.

Les paramètres de synthèse pouvant permettre de contrôler la porosité de l'aggloméré adsorbant zéolithique de l'invention sont également connus de l'homme du métier. De manière générale, ces paramètres comprennent, de manière non limitative le taux de liant, le type d'agglomération (par extrusion, atomisation, granulation, etc.), le taux d'humidité, la nature du liant, les conditions de zéolithisation (température, durée, pH de la solution alcaline de zéolithisation, durée, mode d'agitation, taux de cisaillement, pression et autre).

Selon un mode de réalisation préféré, la synthèse de l'aggloméré adsorbant zéolithique de la présente invention ne comprend pas l'ajout d'agent porogène, la présence d'un agent porogène pouvant entraîner notamment une dégradation de la cristallinité.

Il est également possible de préparer lesdits éléments cristallins par synthèse par ensemencement et/ou par ajustement des conditions opératoires de synthèse tels que le rapport $SiO_2/Al_2O_3$, la teneur en sodium et l'alcalinité du mélange de synthèse.

La synthèse de zéolithe de type FAU se fait généralement en milieu sodique (cation $Na^+$). Les éléments cristallins de zéolithe FAU ainsi obtenus comprennent majoritairement, voire exclusivement des cations sodium. On ne sortirait toutefois pas du cadre de l'invention en utilisant des éléments cristallins ayant subi un ou plusieurs échanges cationiques, entre la synthèse sous forme sodique.

La taille des cristaux de zéolithe FAU utilisés à l'étape a) et des éléments cristallins de zéolithe FAU dans les agglomérés selon l'invention est mesurée par observation au microscope électronique à balayage (MEB). Comme indiqué précédemment, le diamètre moyen des cristaux est compris entre 100 nm et 1200 nm, de préférence encore compris entre 400 nm et 1200 nm, plus préférentiellement entre 500 nm et 1200 nm, de manière tout à fait préférée entre 550 nm et 1200 nm, très avantageusement entre 600 nm et 1200 nm, bornes incluses.

Cette observation MEB permet également de confirmer la présence de phase non zéolithique comprenant par exemple du liant résiduel (non converti lors de l'étape de zéolithisation) ou toute autre phase amorphe dans les agglomérés.

Dans le présent document, on emploie l'appellation « diamètre moyen en nombre » ou bien « taille », notamment pour les éléments cristallins de zéolithe et pour les adsorbants zéolithiques. La méthode de mesure de ces grandeurs est explicitée plus loin dans la description.

L'agglomération et la mise en forme (étape a) peuvent être réalisées selon toutes les techniques connues de l'homme de l'art, telles qu'extrusion, compactage, agglomération sur assiette granulatrice, tambour granulateur, atomisation et autres.

Les proportions de liant d'agglomération (voir définition plus loin) et de zéolithe mises en oeuvre sont typiquement celles de l'art antérieur, c'est-à-dire de 5 parties à 20 parties en poids de liant pour 95 parties à 80 parties en poids de zéolithe.

Les agglomérés issus de l'étape a), qu'ils soient sous forme de billes, d'extrudés ou autres, ont en général un diamètre moyen en nombre, ou leur longueur (plus grande dimension lorsqu'ils ne sont pas sphériques), comprise entre 100 $\mu$m et 1000 $\mu$m, de préférence entre 100 $\mu$m et 600 $\mu$m, de préférence encore 200 $\mu$m et 550 $\mu$m, bornes incluses.

À l'issue de l'étape a) les agglomérés les plus fins peuvent être éliminés par cyclonage et/ou tamisage et/ou les agglomérés trop gros par tamisage ou concassage, dans le cas d'extrudés, par exemple.

Le liant d'agglomération utilisé dans l'étape a) comprend, et de préférence consiste en, une argile ou un mélange d'argiles. Ces argiles sont de préférence choisies parmi les kaolins, kaolinites, nacrites, dickites, halloysites, attapulgites, sépiolites, montmorillonites, bentonites, illites et métakaolins, ainsi que les mélanges de deux ou plusieurs d'entre elles en toutes proportions.

Dans le cas de l'étape de zéolithisation le liant d'agglomération mis en oeuvre à l'étape a) contient au moins 80% de préférence, au moins 90%, de préférence encore au moins 95%, plus particulièrement au moins 96%, en poids, d'au moins une argile zéolithisable et peut également contenir d'autres liants minéraux tels que bentonite, attapulgite, et autres. Par argile zéolithisable, on entend une argile ou un mélange d'argiles qui sont susceptibles d'être converties en matière zéolithique, le plus souvent par action d'une solution basique alcaline. L'argile zéolithisable appartient en général à la famille des kaolins (tels que par exemple kaolinites, nacrites, dickites, halloysites) et/ou des métakaolins.

Parmi les additifs éventuellement mis en oeuvre à l'étape a), on peut trouver une source de silice de tout type connu de l'homme du métier, spécialiste de la synthèse de zéolithes, par exemple de la silice colloïdale, des diatomées, de la perlite, des cendres de calcination (« fly ash » en langue anglaise), du sable, ou toute autre forme de silice solide.

Lors de l'étape a), outre les éléments cristallins de zéolithe FAU et le liant, d'autres additifs peuvent également être ajoutés, par exemple des additifs destinés à faciliter l'agglomération ou à améliorer le durcissement, ainsi que d'autres additifs connus de l'homme du métier.

En particulier si le liant d'agglomération contient une ou plusieurs argiles zéolithisables, la calcination permet de transformer l'argile zéolithisable, typiquement le kaolin, en méta-kaolin qui peut après être converti en zéolithe lors de l'étape de zéolithisation (étape b)). Le principe en est exposé dans « Zeolite Molecular Sieves » de D. W. Breck, John Wiley and Sons, New York, (1973), p. 314-315.

La zéolithisation du liant d'agglomération est pratiquée selon toute méthode maintenant bien connue de l'homme du métier et peut par exemple être réalisée par immersion du produit issu de l'étape a) dans une solution basique alcaline, en général aqueuse, par exemple une solution aqueuse d'hydroxyde de sodium et/ou d'hydroxyde de potassium.

En règle générale, la concentration de la solution alcaline de zéolithisation est de préférence comprise entre 0,5 M et 5 M. La zéolithisation s'opère de préférence à chaud, à une température supérieure à la température ambiante, et typiquement à des températures de l'ordre de 80°C à 100°C. La durée du processus de zéolithisation est généralement comprise entre quelques dizaines de minutes et quelques heures, de préférence entre environ 1 heure et 8 heures.

De manière préférée et afin d'assurer une zéolithisation complète du liant sans altérer la cristallinité des cristaux de zéolithe présents on préférera mettre en contact les adsorbants avec une solution de soude froide puis effectuer une montée en température graduelle jusqu'à la température de 80°C-100°C.

De la même façon, la concentration en soude pourra être maintenue à la même concentration ou pourra être augmentée de façon graduelle afin de maintenir une cristallinité maximale des cristaux initiaux et assurer une conversion maximale du liant zéolithisable.

Les étapes d'échange(s) cationique(s) c) et d) s'effectuent selon les méthodes classiques connues de l'homme du métier, et le plus souvent par mise en contact des agglomérés issus de l'étape a) avec un sel de baryum et/ou de potassium, tel que le chlorure de baryum ($BaCl_2$) et/ou de potassium (KCl), en solution aqueuse à une température comprise entre la température ambiante et 100°C, et de préférence comprise entre 80°C et 100°C afin d'obtenir rapidement des teneurs en baryum, exprimées en oxyde de baryum, élevées, i.e. de préférence supérieures à 10%, de préférence supérieures à 15%, de manière très préférée supérieures à 20%, de manière encore plus préférée supérieures à 23%, voire supérieures à 33% en poids par rapport à la masse totale de l'adsorbant.

Avantageusement, la teneur en baryum, exprimée en oxyde de baryum, est comprise entre 23% et 42%, et typiquement entre 30% et 40%, bornes incluses, en poids par rapport au poids total de l'adsorbant. On préfère opérer avec un large excès d'ions baryum par rapport aux cations de la zéolithe que l'on souhaite échanger, typiquement un excès de l'ordre de 10 à 12, avantageusement en procédant par échanges successifs.

L'échange éventuel au potassium (étape d) peut être pratiqué avant et/ou après l'échange au baryum (étape c). Comme indiqué précédemment, il est également possible d'agglomérer à l'étape a) des éléments cristallins de zéolithe FAU contenant déjà des ions baryum ou potassium ou baryum et potassium (pré-échange des cations présents dans la zéolithe de type FAU de départ, typiquement cations sodium, par des ions baryum ou potassium ou baryum et potassium avant l'étape a) et s'affranchir (ou non) des étapes c) et/ou d).

Après la ou les étape(s) d'échange(s) cationique(s), on procède ensuite à un lavage, généralement et de préférence à l'eau, puis d'un séchage de l'aggloméré ainsi obtenu.

L'activation qui suit le séchage, est conduite de manière classique, selon les méthodes connues de l'homme du métier, par exemple à une température en général comprise entre 100°C et 400°C, de préférence entre 200°C et 300°C pendant une durée déterminée en fonction de la teneur en eau et de la perte au feu souhaitées, typiquement de 1 heure à 6 heures.

TECHNIQUES DE CARACTERISATION

Granulométrie des cristaux de zéolithe :

L'estimation du diamètre moyen en nombre des éléments (i.e. cristaux) de zéolithe de type FAU utilisées à l'étape a) et des éléments (i.e. cristaux) de zéolithe X contenus dans les agglomérés est réalisée par observation au microscope électronique à balayage (MEB).

Afin d'estimer la taille des particules (i.e. cristaux) de zéolithe sur les échantillons, on effectue un ensemble de clichés à un grossissement d'au moins 5000. On mesure ensuite le diamètre d'au moins 200 particules à l'aide d'un logiciel dédié, par exemple le logiciel Smile View de l'éditeur LoGraMi. La précision est de l'ordre de 3%. La mesure de l'histogramme constitué à partir des dites mesures de diamètre permet en même temps la détermination de la déviation standard $\sigma$ de sa distribution.

Analyse chimique des agglomérés adsorbants zéolithiques - rapport Si/Al et taux d'échange :

Une analyse chimique élémentaire du produit final obtenu à l'issue des étapes a) à f) décrites précédemment, peut être réalisée selon différentes techniques analytiques connues de l'homme du métier. Parmi ces techniques, on peut citer la technique d'analyse chimique par fluorescence de rayons X telle que décrite dans la norme NF EN ISO 12677 : 2011 sur un spectromètre dispersif en longueur d'onde (WDXRF), par exemple Tiger S8 de la société Bruker.

La fluorescence X est une technique spectrale non destructive exploitant la photoluminescence des atomes dans le domaine des rayons X, pour établir la composition élémentaire d'un échantillon. L'excitation des atomes généralement par un faisceau de rayons X ou par bombardement avec des électrons, génère des radiations spécifiques après retour à l'état fondamental de l'atome. Le spectre de fluorescence X a l'avantage de dépendre très peu de la combinaison chimique de l'élément, ce qui offre une détermination précise, à la fois quantitative et qualitative. On obtient de manière classique après étalonnage pour chaque oxyde une incertitude de mesure inférieure à 0,4% en poids.

Ces analyses chimiques élémentaires permettent à la fois de vérifier le rapport atomique Si/Al de la zéolithe utilisée au sein de l'aggloméré et le rapport atomique Si/Al du produit final obtenu à l'issue des étapes a) à f) décrites précédemment, et de vérifier la qualité de l'échange ionique décrit à l'étape c) et à l'étape optionnelle d). Dans la description de la présente invention, l'incertitude de mesure du rapport atomique Si/Al est de $\pm$ 5%.

La qualité de l'échange ionique est liée au nombre de mole d'oxyde de sodium, $Na_2O$, restant dans l'aggloméré zéolithique après échange. Plus précisément, le taux d'échange par les ions baryum est estimé en évaluant le rapport entre le nombre de moles d'oxyde de baryum, BaO, et le nombre de moles de l'ensemble (BaO + Na$_2$O). De même, le taux d'échange par les ions baryum et/ou potassium est estimé en évaluant le rapport entre le nombre de moles de l'ensemble oxyde de baryum + oxyde de potassium (BaO + K$_2$O) et le nombre de moles de l'ensemble (BaO + K$_2$O + Na$_2$O). Il est à noter que les teneurs en différents oxydes sont donnés, en pourcentage en poids par rapport au poids total de l'adsorbant zéolithique anhydre.

Granulométrie des adsorbants zéolithiques :

La détermination du diamètre moyen en nombre des adsorbants zéolithiques obtenus à l'issus de l'étape a) d'agglomération et de mise en forme est effectuée par analyse de la distribution granulométrique d'un échantillon d'aggloméré par imagerie selon la norme ISO 13322-2:2006, en utilisant un tapis roulant permettant à l'échantillon de passer devant l'objectif de la caméra.

Le diamètre moyen en nombre est ensuite calculé à partir de la distribution granulométrique en appliquant la norme ISO 9276-2:2001. Dans le présent document, on emploie l'appellation « diamètre moyen en nombre » ou bien « taille » pour les agglomérés zéolithiques. La précision est de l'ordre de 0,01 mm pour la gamme de taille d'agglomérés de l'invention.

Résistance mécanique des adsorbants zéolithiques :

La résistance à l'écrasement d'un lit d'adsorbants zéolithiques tels que décrits dans la présente invention est caractérisée selon la méthode Shell série SMS1471-74 (Shell Method Series SMS1471-74 « Détermination of Bulk Crushing Strength of Catalysts. Compression-Sieve Method »), associée à l'appareil « BCS Tester » commercialisé par la société Vinci Technologies. Cette méthode, initialement destinée à la caractérisation de catalyseurs de 3 mm à 6 mm est basée sur l'utilisation d'un tamis de 425 μm qui va permettre notamment de séparer les fines créées lors de l'écrasement. L'utilisation d'un tamis de 425 μm reste adaptée pour des particules de diamètre supérieur à 1,6 mm, mais doit être adaptée selon la granulométrie des agglomérés que l'on cherche à caractériser.

Les agglomérés de la présente invention, généralement sous forme de billes ou d'extrudés, ont en général un diamètre moyen en nombre ou une longueur, i.e. plus grande dimension dans le cas des agglomérés non sphériques, comprise entre 0,2 mm et 2 mm, et en particulier comprise entre 0,2 mm et 0,8 mm et de préférence entre 0,4 mm et 0,65 mm, bornes incluses. Par conséquent, un tamis de 100 μm est utilisé à la place du tamis de 425 μm mentionné dans la méthode Shell standard SMS1471-74.

Le protocole de mesure est le suivant : un échantillon de 20 cm$^3$ d'adsorbants agglomérés, préalablement tamisé avec le tamis adapté (100 μm) et préalablement séché à l'étuve pendant au moins 2 heures à 250°C (au lieu de 300°C mentionné dans la méthode Shell standard SMS1471-74), est placé dans un cylindre métallique de section interne connue. Une force croissante est imposée par paliers sur cet échantillon par l'intermédiaire d'un piston, à travers un lit de 5 cm$^3$ de billes d'acier afin de mieux répartir la force exercée par le piston sur les agglomérés d'adsorbants (utilisation de billes de 2 mm de diamètre pour des particules de forme sphérique de diamètre strictement inférieur à 1,6 mm). Les fines obtenues aux différents paliers de pression sont séparées par tamisage (tamis adapté de 100 μm) et pesées.

La résistance à l'écrasement en lit est déterminée par la pression en mégaPascal (MPa) pour laquelle la quantité de fines cumulées passant à travers le tamis s'élève à 0,5% pondéral de l'échantillon. Cette valeur est obtenue en traçant sur un graphique la masse de fines obtenue en fonction de la force appliquée sur le lit d'adsorbant et en interpolant à 0,5 % massique de fines cumulées. La résistance mécanique à l'écrasement en lit est typiquement comprise entre quelques centaines de kPa et quelques dizaines de MPa et généralement comprise entre 0,3 MPa et 3,2 MPa. La précision est de manière classique inférieure à 0,1 MPa.

Phase non zéolithique des adsorbants zéolithiques :

Le taux de phase non zéolithique PNZ, par exemple liant résiduel non zéolithisé ou toute autre phase amorphe, après zéolithisation est calculé selon l'équation suivante :

$$PNZ = 100 - \Sigma\ (PZ)$$

où PZ représente la somme des quantités des fractions zéolithiques X au sens de l'invention.

La quantité des fractions zéolithiques X (taux de cristallinité) est mesurée par analyse par diffraction de rayons X, connue de l'homme du métier sous l'acronyme DRX. Cette analyse est réalisée sur un appareil de la marque Bruker, puis la quantité des fractions zéolithiques X est évaluée au moyen du logiciel TOPAS de la société Bruker.

Volume microporeux :

La cristallinité des agglomérés est également évaluée par mesure de leur volume microporeux en le comparant à celui d'une référence appropriée (zéolithe 100% cristalline dans des conditions de traitements cationiques identiques ou zéolithe théorique). Ce volume microporeux est déterminé à partir de la mesure de l'isotherme d'adsorption de gaz, tel que l'azote, à sa température de liquéfaction.

Préalablement à l'adsorption, l'adsorbant zéolithique est dégazé entre 300°C et 450°C pendant une durée comprise entre 9 heures et 16 heures, sous vide (P < $6,7.10^{-4}$ Pa). La mesure de l'isotherme d'adsorption d'azote à 77 K est ensuite effectuée sur un appareil de type ASAP 2020 M de Micromeritics, en prenant au moins 35 points de mesure à des pressions relatives de rapport PIPa compris entre 0,002 et 1.

Volume total des macropores et des mésopores, et porosité de grain :

Les volumes macroporeux $V_{ma}$ et mésoporeux $V_{me}$, la densité de grain $d_g$ et la porosité $\varepsilon_p$, de type macroporosité et mésoporosité, sont mesurés par porosimétrie par intrusion de mercure. Un porosimètre à mercure type Autopore® 9500 de Micromeritics est utilisé pour analyser la répartition du volume poreux contenu dans les macropores et dans les mésopores.

La méthode expérimentale, décrite dans le manuel opératoire de l'appareil faisant référence à la norme ASTM D4284-83, consiste à placer un échantillon d'adsorbant (adsorbant zéolithique sous forme d'agglomérés à mesurer) (de perte au feu connue) préalablement pesé, dans une cellule du porosimètre, puis, après un dégazage préalable (pression d'évacuation de 30 μm de mercure pendant au moins 10 min), à remplir la cellule avec du mercure à une pression donnée (0,0036 MPa), et ensuite à appliquer une pression croissante par palier jusqu'à 400 MPa afin de faire pénétrer progressivement le mercure dans le réseau poreux de l'échantillon, en prenant au moins 15 paliers de pression jusqu'à 0,2 MPa, et en appliquant ensuite des incréments de 0,1 MPa jusqu'à 1 MPa, puis 0,5 MPa jusqu'à 10 MPa, puis 2 MPa jusqu'à 30 MPa, puis 5 MPa jusqu'à 180 MPa, et enfin 10 MPa jusqu'à 400 MPa.

La relation entre la pression appliquée et la dimension caractéristique du seuil d'entrée de pore (correspondant à un diamètre apparent de pore) est établie en utilisant l'équation de Laplace-Young et en supposant une ouverture de pore cylindrique, un angle de contact entre le mercure et la paroi des pores de 140° et une tension de surface du mercure de 485 dynes $cm^{-1}$. Les incréments de volume $\Delta V_i$ de mercure introduit à chaque palier de pression $P_i$ sont enregistrés, ce qui permet ensuite de tracer le volume cumulé de mercure introduit en fonction de la pression appliquée $V(P_i)$, ou en fonction du diamètre apparent des pores $V(l_i)$. On fixe à 0,2 MPa la valeur à partir de laquelle le mercure remplit tous les vides inter-granulaires, et on considère qu'au-delà le mercure pénètre dans les pores de l'adsorbant. Le volume de grain $V_g$ est alors calculé en soustrayant le volume cumulé de mercure à cette pression (0,2 MPa) au volume de la cellule du porosimètre, et en divisant cette différence par la masse de l'adsorbant équivalent anhydre, c'est-à-dire la masse dudit matériau corrigée de la perte au feu. La densité de grain $d_g$ est l'inverse du volume de grain $V_g$ défini précédemment.

Le volume macroporeux $V_{ma}$ de l'adsorbant est défini comme étant le volume cumulé de mercure introduit à une pression comprise entre 0,2 MPa et 30 MPa, correspondant au volume contenu dans les pores de diamètre apparent supérieur à 50 nm. Le volume mésoporeux $V_{me}$ de l'adsorbant est défini comme étant le volume cumulé de mercure introduit à une pression comprise entre 30 MPa et 400 MPa. La méthode de mesure du volume poreux par intrusion de mercure ne permettant pas d'accéder au volume microporeux, le volume poreux total $V_{tot}$ tel que mesuré par intrusion de mercure, correspond à la somme des volumes macroporeux $V_{ma}$ et mésoporeux $V_{me}$.

Dans le présent document, les volumes macroporeux et mésoporeux $V_{ma}$ et $V_{me}$, ainsi que leur somme (volume poreux total $V_{tot}$), des adsorbants zéolithiques, exprimés en $cm^3 \, g^{-1}$, sont ainsi mesurés par porosimétrie par intrusion de mercure et rapportés à la masse de l'échantillon en équivalent anhydre, c'est-à-dire la masse dudit adsorbant corrigée de la perte au feu. La densité de grain $d_g$ est exprimée en $g \, cm^{-3}$ et se réfère à la masse de l'échantillon en équivalent anhydre.

La porosité de grain $\varepsilon_p$ de type macroporosité et mésoporosité, est le produit de la densité de grain $d_g$ par la somme des volumes macroporeux et mésoporeux $V_{ma}$ et $V_{me}$ :

$$\varepsilon p \;=\; dg \times (Vma \,+\, Vme)$$

Perte au feu des adsorbants zéolithiques :

La perte au feu est déterminée en atmosphère oxydante, par calcination de l'échantillon à l'air à une température de 900°C $\pm$ 25°C, en suivant le mode opératoire décrit dans la norme NF EN 196-2 (avril 2006). L'écart type de mesure est inférieur à 0,1%.

Exemples

Exemple 1 : Préparation des agglomérés

On prépare quatre adsorbants (Agglomérés 1, 3, 4, selon l'invention, et Aggloméré 2, comparatif) comme décrit ci-dessous, à partir d'une poudre de zéolithe faujasite de type X, dont la taille moyenne des cristaux est de 0,6 $\mu$m. Les déviations standard respectives de ces cristaux sont de 0,25 $\mu$m, 0,30 $\mu$m 0,35 $\mu$m et 0,50 $\mu$m.

Préparation de l'Aggloméré 1 (selon l'invention)

On prépare un mélange homogène et on agglomère 800 g de cristaux de zéolithe, de déviation standard de 0,25 $\mu$m, avec 160 g de kaolin (exprimés en équivalent calciné) et 60 g de silice colloïdale vendue sous la dénomination commerciale Klebosol™ 30N50 (contenant 30% en poids de $SiO_2$ et 0,5% en poids de $Na_2O$) avec la quantité d'eau qui permet l'extrusion du mélange. Les extrudés sont séchés, puis calcinés à 550°C (cuisson de l'argile) sous courant d'azote pendant 2 heures, et enfin concassés de manière à récupérer des agglomérés dont le diamètre moyen en nombre est égal à 0,5 mm.

Les agglomérés obtenus comme décrit ci-dessus (20 g) sont placés dans un réacteur en verre muni d'une double enveloppe régulée à une température de 85°C $\pm$ 1°C puis on ajoute 250 mL d'une solution aqueuse d'hydroxyde de sodium de concentration de 1 M, et on laisse le milieu réactionnel sous agitation pendant une durée de 5 heures.

On procède ensuite au lavage des agglomérés en 3 opérations successives de lavage à l'eau suivi de la vidange du réacteur. On s'assure de l'efficacité du lavage en mesurant le pH final des eaux de lavage qui est compris entre 10,0 et 10,5.

Les agglomérés sont échangés, par mise en contact avec une solution de chlorure de baryum 0,5 M à 95°C en 4 étapes. À chaque étape, le rapport volume de solution sur masse de solide est de 20 mL/g et l'échange est poursuivi pendant 4 heures à chaque fois. Entre chaque échange, le solide est lavé plusieurs fois de manière à le débarrasser des excédents de sel. Les agglomérés sont ensuite séchés à 80°C pendant 2 heures et enfin activés à 250°C pendant 2 heures sous courant d'azote.

Préparation de l'Aggloméré 2 (comparatif)

On prépare un mélange homogène et on agglomère 800 g de cristaux de zéolithe de déviation standard de 0,50 $\mu$m, avec 145 g de kaolin (exprimés en équivalent calciné) et 55 g de silice colloïdale vendue sous la dénomination commerciale Klebosol™ 30N50 (contenant 30% en poids de $SiO_2$ et 0,5% en poids de $Na_2O$) avec la quantité d'eau qui permet l'extrusion du mélange. Les extrudés sont séchés, puis calcinés à 550°C (cuisson de l'argile) sous courant d'azote pendant 2 heures, et enfin concassés de manière à récupérer des agglomérés dont le diamètre moyen en nombre est égal à 0,5 mm.

Les agglomérés obtenus comme décrit ci-dessus (20 g) sont placés dans un réacteur en verre muni d'une double enveloppe régulée à une température de 95°C $\pm$ 1°C puis on ajoute 250 mL d'une solution aqueuse d'hydroxyde de sodium de concentration de 1,25 M, et on laisse le milieu réactionnel sous agitation pendant une durée de 4 heures.

On procède ensuite au lavage des agglomérés en 3 opérations successives de lavage à l'eau suivi de la vidange du réacteur. On s'assure de l'efficacité du lavage en mesurant le pH final des eaux de lavage qui est compris entre 10,0 et 10,5.

Les agglomérés sont échangés, par mise en contact avec une solution de chlorure de baryum 0,5 M à 95°C en 4 étapes. À chaque étape, le rapport volume de solution sur masse de solide est de 20 mL $g^{-1}$ et l'échange est poursuivi pendant 4 heures à chaque fois. Entre chaque échange, le solide est lavé plusieurs fois de manière à le débarrasser des excédents de sel. Les agglomérés sont ensuite séchés à 80°C pendant 2 heures et enfin activés à 250°C pendant 2 heures sous courant d'azote.

Préparation de l'Aggloméré 3 (selon l'invention)

On prépare un mélange homogène et on agglomère 800 g de cristaux de zéolithe, de déviation standard de 0,30 $\mu$m, avec 160 g de kaolin (exprimés en équivalent calciné) et 60 g de silice colloïdale vendue sous la dénomination commerciale Klebosol™ 30N50 (contenant 30% en poids de $SiO_2$ et 0,5% en poids de $Na_2O$) avec la quantité d'eau qui permet l'extrusion du mélange. Les extrudés sont séchés, puis calcinés à 550°C (cuisson de l'argile) sous courant d'azote pendant 2 heures, et enfin concassés de manière à récupérer des agglomérés dont le diamètre moyen en nombre est égal à 0,5 mm.

Les agglomérés obtenus comme décrit ci-dessus (20 g) sont placés dans un réacteur en verre muni d'une double enveloppe régulée à une température de 90°C ± 1°C puis on ajoute 250 mL d'une solution aqueuse d'hydroxyde de sodium de concentration de 0,9 M, et on laisse le milieu réactionnel sous agitation pendant une durée de 6 heures.

On procède ensuite au lavage des agglomérés en 3 opérations successives de lavage à l'eau suivi de la vidange du réacteur. On s'assure de l'efficacité du lavage en mesurant le pH final des eaux de lavage qui est compris entre 10,0 et 10,5.

Les agglomérés sont échangés, par mise en contact avec une solution de chlorure de baryum 0,5 M à 95°C en 4 étapes. À chaque étape, le rapport volume de solution sur masse de solide est de 20 mL/g et l'échange est poursuivi pendant 4 heures à chaque fois. Entre chaque échange, le solide est lavé plusieurs fois de manière à le débarrasser des excédents de sel. Les agglomérés sont ensuite séchés à 80°C pendant 2 heures et enfin activés à 250°C pendant 2 heures sous courant d'azote.

Préparation de l'Aggloméré 4 (selon l'invention)

On prépare un mélange homogène et on agglomère 800 g de cristaux de zéolithe, de déviation standard de 0,35 $\mu$m, avec 150 g de kaolin (exprimés en équivalent calciné) et 58 g de silice colloïdale vendue sous la dénomination commerciale Klebosol™ 30N50 (contenant 30% en poids de $SiO_2$ et 0,5% en poids de $Na_2O$) avec la quantité d'eau qui permet l'extrusion du mélange. Les extrudés sont séchés, puis calcinés à 550°C (cuisson de l'argile) sous courant d'azote pendant 2 heures, et enfin concassés de manière à récupérer des agglomérés dont le diamètre moyen en nombre est égal à 0,5 mm.

Les agglomérés obtenus comme décrit ci-dessus (20 g) sont placés dans un réacteur en verre muni d'une double enveloppe régulée à une température de 90°C ± 1°C puis on ajoute 250 mL d'une solution aqueuse d'hydroxyde de sodium de concentration de 1,15 M, et on laisse le milieu réactionnel sous agitation pendant une durée de 5 heures.

On procède ensuite au lavage des agglomérés en 3 opérations successives de lavage à l'eau suivi de la vidange du réacteur. On s'assure de l'efficacité du lavage en mesurant le pH final des eaux de lavage qui est compris entre 10,0 et 10,5.

Les agglomérés sont échangés, par mise en contact avec une solution de chlorure de baryum 0,5 M à 95°C en 4 étapes. À chaque étape, le rapport volume de solution sur masse de solide est de 20 mL/g et l'échange est poursuivi pendant 4 heures à chaque fois. Entre chaque échange, le solide est lavé plusieurs fois de manière à le débarrasser des excédents de sel. Les agglomérés sont ensuite séchés à 80°C pendant 2 heures et enfin activés à 250°C pendant 2 heures sous courant d'azote.

Caractéristiques des Agglomérés 1 à 4

La résistance mécanique (REL) des agglomérés est caractérisée par les techniques de caractérisation décrites ci-dessus, et on obtient, ainsi une valeur de 3,2 MPa pour l'Aggloméré 1, et une valeur de 3,1 MPa pour l'Aggloméré 2, une valeur de 2,9 MPa pour l'Aggloméré 3 et une valeur de 3,1 MPa pour l'Aggloméré 4.

Les valeurs de porosité de grain $\varepsilon_p$ et les déviations standard $\sigma$ des cristaux dans les agglomérés finaux, et telles que mesurées pour les Agglomérés 1 à 4 sont données dans le Tableau 1 ci-dessous.

L'Aggloméré 1, à base de cristaux de BaX, présente une taille moyenne de cristaux mesurée sur l'aggloméré final de 0,79 $\mu$m, l'Aggloméré 2, à base de cristaux de BaX, présente une taille moyenne de cristaux mesurée sur l'aggloméré final de 0,76 $\mu$m, l'Aggloméré 3, à base de cristaux de BaX, présente une taille moyenne de cristaux mesurée sur l'aggloméré final de 0,77 $\mu$m, et l'Aggloméré 4, à base de cristaux de BaX, présente une taille moyenne de cristaux mesurée sur l'aggloméré final de 0,76 $\mu$m.

Les taux d'échange baryum des Agglomérés 1 à 4 calculés à partir des analyses élémentaires des oxydes de baryum et de sodium par fluorescence de rayons X, comme décrit dans les techniques de caractérisation, est de 99,0%.

La teneur en BaO dans les agglomérés 1 à 4 est égale à 36,2% en poids.

La perte au feu mesurée comme décrit précédemment est de 5,2% ± 0,1%.

Exemple 2 : mis en oeuvre dans un procédé de séparation

Mise en oeuvre des Agglomérés 1, 3 et 4

Les Agglomérés 1, 3 et 4 sont utilisés pour la séparation de paraxylène en lit mobile simulé.

**EP 4 076 731 B1**

On utilise une unité fonctionnant en lit mobile simulé constituée de 24 lits, de longueur 1,1 m, avec une injection de charge, une injection de désorbant, un soutirage d'extrait et un soutirage de raffinat. Les lits sont répartis en 4 zones chromatographiques selon la configuration : 5 / 9 / 7 / 3.

La charge est composée de 50% de para-xylène, de 14,5% d'ortho-xylène, de 30,6% de méta-xylène et de 4,9% d'éthylbenzène. Le désorbant est du *para*-diéthylbenzène. La température est de 175°C, et la pression de 15 bars. La teneur en eau est de 95 ppm (poids).

Les productivités obtenues sont présentées dans le Tableau 1 ci-dessous. La vitesse superficielle linéaire en zone 3 est de 1,63 cm s$^{-1}$.

Mise en oeuvre de l'Aggloméré 2

L'Aggloméré 2 est utilisé pour la séparation de paraxylène en lit mobile simulé.

On utilise une unité fonctionnant en lit mobile simulé constituée de 24 lits, de longueur 1,1 m, avec une injection de charge, une injection de désorbant, un soutirage d'extrait et un soutirage de raffinat. Les lits sont répartis en 4 zones chromatographiques selon la configuration : 5 / 9 / 7 / 3.

La charge est composée de 50% de *para*-xylène, de 14,5% d'*ortho*-xylène, de 30,6% de *méta*-xylène et de 4,9% d'éthylbenzène. Le désorbant est du *para*-diéthylbenzène. La température est de 175°C, et la pression de 15 bars. La teneur en eau est de 95 ppm (poids).

La productivité est de 186 kg de *para*-xylène m$^{-3}$ h$^{-1}$. La vitesse superficielle linéaire en zone 3 est de 1,63 cm s$^{-1}$.

Les résultats de productivité des quatre adsorbants agglomérés sont donnés dans le Tableau 1 ci-dessous.

Tableau 1

| | Porosité de grain $\varepsilon_p$ (%) | Déviation standard $\sigma$ ($\mu m$) | Productivité (kg para-xylène m$^{-3}$ h$^{-1}$) |
|---|---|---|---|
| **Aggloméré 1** (selon l'invention) | 35 | 0,21 | 220 |
| **Aggloméré 2** (comparatif) | 24 | 0,32 | 186 |
| **Aggloméré 3** (selon l'invention) | 32 | 0.28 | 214 |
| **Aggloméré 4** (selon l'invention) | 38 | 0,15 | 223 |

On montre bien que les agglomérés selon l'invention permettent d'obtenir un gain en productivité significatif sur le procédé de production de para-xylène.

**Revendications**

**1.** Adsorbant zéolithique aggloméré, comprenant au moins une zéolithe faujasite de type FAU-X de rapport atomique Si/Al compris entre 1,00 et 1,50 bornes incluses et comprenant du baryum et éventuellement du potassium, caractérisé d'une part en ce que la porosité de grain dudit adsorbant est comprise entre 25% et 45 %, de préférence entre 30% et 45 %, de préférence encore entre 32% et 45% et de manière très préférée entre 35% et 45%, de manière particulièrement avantageuse entre 36% et 45%, bornes incluses, et d'autre part en ce que la déviation standard $\sigma$ de la distribution de taille de cristaux dans ledit aggloméré est inférieure à 0,30 $\mu m$, de préférence comprise entre 0,05 $\mu m$ et 0,30 $\mu m$, de préférence encore entre 0,05 $\mu m$ et 0,28 $\mu m$, et de manière tout à fait préférée entre 0,1 $\mu m$ et 0,28 $\mu m$, de préférence entre toutes entre 0,1 $\mu m$ et 0,25 $\mu m$, bornes incluses, ledit adsorbant comprenant des cristaux de zéolithe de diamètre moyen en nombre compris entre 100 nm et 1200 nm, bornes incluses, et la fraction massique de zéolithe FAU étant supérieure ou égale à 80% par rapport au poids total d'absorbant, dans lequel la porosité et la déviation standard de la distribution des tailles de cristaux sont tels que mesurés dans la description.

**2.** Adsorbant zéolithique aggloméré selon la revendication 1 dans lequel ledit adsorbant comprend des cristaux de zéolithe de diamètre moyen compris entre 400 nm et 1200 nm, plus préférentiellement entre 500 nm et 1200 nm, de manière tout à fait préférée entre 550 nm et 1200 nm, très avantageusement entre 600 nm et 1200 nm, bornes incluses.

3. Adsorbant zéolithique aggloméré selon l'une des revendications 1 ou 2, **caractérisé en ce qu'**il se présente sous la forme de billes de diamètre moyen compris entre 100 $\mu$m et 1000 $\mu$m, de préférence entre 100 $\mu$m et 600 $\mu$m, de préférence encore 200 $\mu$m et 550 $\mu$m, bornes incluses.

4. Adsorbant zéolithique aggloméré selon l'une des revendications 1 à 3 dans lequel ladite au moins une zéolithe FAU-X présente un rapport atomique Si/Al compris entre 1,05 et 1,50, de préférence entre 1,05 et 1,40, bornes incluses et de manière encore plus préférée entre 1,10 et 1,40 bornes incluses.

5. Adsorbant zéolithique aggloméré selon l'une des revendications 1 à 4, **caractérisé en ce qu'**il contient du baryum en une teneur, exprimée en poids d'oxyde de baryum (BaO) supérieure à 10%, de préférence supérieure à 15%, de manière très préférée supérieure à 20%, de manière encore plus préférée supérieure à 23 %, voire supérieure à 33% en poids par rapport à la masse totale de l'adsorbant, et avantageusement, la teneur en baryum est comprise entre 23% et 42%, et typiquement entre 30% et 40%, bornes incluses, en poids par rapport au poids total de l'adsorbant.

6. Adsorbant zéolithique aggloméré selon l'une des revendications 1 à 5, **caractérisé en ce qu'**il contient du potassium en une teneur, exprimée en poids d'oxyde de potassium $K_2O$ inférieure à 25%, de préférence comprise entre 0 et 20%, de manière encore plus préférée comprise entre 0 et 15%, bornes incluses, en poids par rapport à la masse totale de l'adsorbant.

7. Adsorbant zéolithique aggloméré selon l'une quelconque des revendications précédentes, dans lequel aucune structure zéolithique autre que la structure faujasite, de préférence aucune structure zéolithique autre que la structure faujasite X, n'est détectée par diffraction des rayons X.

8. Adsorbant selon l'une quelconque des revendications précédentes, dans lequel la fraction massique de zéolithe FAU, la zéolithe FAU étant une zéolithe X, est supérieure ou égale à 80% par rapport au poids total d'adsorbant.

9. Utilisation d'un adsorbant selon l'une quelconque des revendications 1 à 8, dans les procédés de :

   - séparation de coupes d'isomères aromatiques en C8 et notamment des xylènes,
   - séparation d'isomères de toluène substitué tels que nitrotoluène, diéthyltoluène, toluènediamine, et autres,
   - séparation des crésols,
   - séparation des alcools polyhydriques.

10. Utilisation selon la revendication 9, pour la séparation de para-xylène à partir de coupes d'isomères aromatiques à 8 atomes de carbone.

11. Procédé de séparation de *para*-xylène à partir de coupes d'isomères aromatiques à 8 atomes de carbone, utilisant comme agent d'adsorption du *para*-xylène, un adsorbant zéolithique aggloméré selon l'une des revendications 1 à 8, en phase liquide ou en phase gazeuse.

12. Procédé de séparation de *para*-xylène à partir de coupes d'isomères d'hydrocarbures aromatiques contenant 8 atomes de carbone, selon la revendication 11, en phase liquide, par adsorption du *para*-xylène en présence d'un désorbant, de préférence choisi parmi le toluène et le *para*-diéthylbenzène.

13. Procédé selon l'une quelconque des revendications 11 ou 12 de type lit mobile simulé, de préférence à contre-courant simulé .

**Patentansprüche**

1. Agglomeriertes Zeolith-Adsorbens, umfassend mindestens einen Faujasit-Zeolith vom Typ FAU-X mit einem Si/Al-Atomverhältnis zwischen 1,00 und 1,50 einschließlich der Grenzwerte und umfassend Barium und gegebenenfalls Kalium, **dadurch gekennzeichnet, dass** einerseits die Kornporosität des Adsorbens zwischen 25 % und 45 %, vorzugsweise zwischen 30 % und 45 %, weiter bevorzugt zwischen 32 % und 45 % und sehr bevorzugt zwischen 35 % und 45 %, besonders bevorzugt zwischen 36 % und 45% einschließlich der Grenzwerte liegt und andererseits die Standardabweichung $\sigma$ der Kristallgrößenverteilung in dem Agglomerat weniger als 0,30 $\mu$m, vorzugsweise zwischen 0,05 $\mu$m und 0,30 $\mu$m, weiter bevorzugt zwischen 0,05 $\mu$m und 0,28 $\mu$m, und vollkommen bevorzugt

zwischen 0,1 μm und 0,28 μm, bevorzugt von allen zwischen 0,1 μm und 0,25 μm einschließlich der Grenzwerte beträgt,

wobei das Adsorbens Zeolithkristalle mit einem zahlenmittleren Durchmesser von zwischen 100 nm und 1200 nm einschließlich der Grenzwerte umfasst und

der Massenanteil an FAU-Zeolith über oder gleich 80 %, bezogen auf das Gesamtgewicht des Adsorbens, beträgt,

wobei die Porosität und die Standardabweichung der Verteilung der Kristallgrößen derart wie in der Beschreibung gemessen sind.

2. Agglomeriertes Zeolith-Adsorbens nach Anspruch 1, wobei das Adsorbens Zeolithkristalle mit einem mittleren Durchmesser zwischen 400 nm und 1200 nm, bevorzugter zwischen 500 nm und 1200 nm, vollkommen bevorzugt zwischen 550 nm und 1200 nm, sehr vorteilhaft zwischen 600 nm und 1200 nm einschließlich der Grenzwerte umfasst.

3. Agglomeriertes Zeolith-Adsorbens nach einem der Ansprüche 1 oder 2, **dadurch gekennzeichnet, dass** es in Form von Kugeln mit einem mittleren Durchmesser zwischen 100 μm und 1000 μm, vorzugsweise zwischen 100 μm und 600 μm, weiter bevorzugt zwischen 200 μm und 550 μm einschließlich der Grenzwerte vorliegt.

4. Agglomeriertes Zeolith-Adsorbens nach einem der Ansprüche 1 bis 3, wobei das mindestens eine FAU-X-Zeolith ein Si/Al-Atomverhältnis zwischen 1,05 und 1,50, vorzugsweise zwischen 1,05 und 1,40 einschließlich der Grenzwerte und noch bevorzugter zwischen 1,10 und 1,40 einschließlich der Grenzwerte aufweist.

5. Agglomeriertes Zeolith-Adsorbens nach einem der Ansprüche 1 bis 4, **dadurch gekennzeichnet, dass** es Barium in einem Gehalt, ausgedrückt in Bariumoxidgewicht (BaO), von über 10%, vorzugsweise von über 15%, sehr bevorzugt von über 20%, noch bevorzugter von über 23 %, sogar von über 33 Gew.-%, bezogen auf die Gesamtmasse des Adsorbens, enthält und vorteilhafterweise der Bariumgehalt zwischen 23 % und 42 % und typischerweise zwischen 30 % und 40 Gew.-% einschließlich der Grenzwerte, bezogen auf das Gesamtgewicht des Adsorbens, liegt.

6. Agglomeriertes Zeolith-Adsorbens nach einem der Ansprüche 1 bis 5, **dadurch gekennzeichnet, dass** es Kalium in einem Gehalt, ausgedrückt in Kaliumoxidgewicht $K_2O$, von unter 25 %, vorzugsweise zwischen 0 bis 20 %, noch bevorzugter zwischen 0 bis 15 Gew.-% einschließlich der Grenzwerte, bezogen auf die Gesamtmasse des Adsorbens, enthält.

7. Agglomeriertes Zeolith-Adsorbens nach einem der vorhergehenden Ansprüche, wobei keine andere Zeolithstruktur als die Faujasitstruktur, vorzugsweise keine andere Zeolithstruktur als die Faujasit-X-Struktur, durch Röntgenstrahlbeugung ermittelt wird.

8. Adsorbens nach einem der vorhergehenden Ansprüche, wobei der Massenanteil an FAU-Zeolith, wobei das FAU-Zeolith ein X-Zeolith ist, über oder gleich 80 %, bezogen auf das Gesamtgewicht des Adsorbens, beträgt.

9. Verwendung eines Adsorbens nach einem der Ansprüche 1 bis 8 in den Verfahren zum:

- Trennen von Schnitten aromatischer C8-Isomere und insbesondere von Xylolen,
- Trennen von Isomeren des substituierten Toluols wie Nitrotoluol, Diethyltoluol, Toluoldiamin und anderen,
- Trennen von Kresolen,
- Trennen von mehrwertigen Alkoholen.

10. Verwendung nach Anspruch 9 zum Trennen von *para*-Xylol aus Schnitten aromatischer Isomere mit 8 Kohlenstoffatomen.

11. Verfahren zum Trennen von *para*-Xylol aus Schnitten aromatischer Isomere mit 8 Kohlenstoffatomen, wobei als Adsorptionsmittel für *para*-Xylol ein agglomeriertes Zeolith-Adsorbens nach einem der Ansprüche 1 bis 8 in der Flüssigphase oder in der Gasphase verwendet wird.

12. Verfahren zum Trennen von *para*-Xylol aus Schnitten aromatischer Kohlenwasserstoffisomere mit 8 Kohlenstoffatomen nach Anspruch 11 in der Flüssigphase durch Adsorption von *para*-Xylol in Gegenwart eines Desorptionsmittels, vorzugsweise ausgewählt aus Toluol und *para*-Diethylbenzol.

**13.** Verfahren nach einem der Ansprüche 11 oder 12 vom Typ simuliertes Wanderbett, vorzugsweise mit simuliertem Gegenstrom.

**Claims**

**1.** Agglomerated zeolitic adsorbent comprising at least one faujasite FAU-X zeolite of Si/Al atomic ratio between 1.00 and 1.50 limits included and comprising barium and optionally potassium, characterized first in that the particle porosity of said absorbent is between 25% and 45%, preferably between 30% and 45%, more preferably between 32% and 45%, further preferably between 35% and 45%, and particularly advantageously between 36% and 45%, limits included, and secondly in that the standard deviation $\sigma$ of crystal size distribution in said agglomerate is less than 0.30 $\mu$m, preferably between 0.05 $\mu$m and 0.30 $\mu$m, more preferably between 0.05 $\mu$m and 0.28 $\mu$m, further preferably between 0.1 $\mu$m and 0.28 $\mu$m, most preferably between 0.1 $\mu$m and 0.25 $\mu$m, limits included? said adsorbent comprises zeolite crystals of number-weighted mean diameter between 100 nm and 1200 nm, limits included,

the weight fraction of FAU zeolite being higher than or equal to 80% relative to the total weight of the adsorbent, wherein the porosity and the standard deviation of crystal size distribution are measured as described in the specification.

**2.** The agglomerated zeolitic adsorbent according to claim 1, wherein said adsorbent comprises zeolite crystals of number-weighted mean diameter between 400 nm and 1200 nm, further preferably between 500 nm and 1200 nm, still further preferably between 550 nm and 1200 nm, most advantageously between 600 nm and 1200 nm, limits included.

**3.** The agglomerated zeolitic adsorbent according to one of claim 1 or 2, **characterized in that** it is in the form of beads of mean diameter between 100 $\mu$m and 1000 $\mu$m, preferably between 100 $\mu$m and 600 $\mu$m, more preferably between 200 $\mu$m and 550 $\mu$m, limits included.

**4.** The agglomerated zeolitic adsorbent according to one of claims 1 to 3, wherein said at least one FAU-X zeolite has a Si/Al atomic ratio of between 1.05 and 1.50, preferably between 1.05 and 1.40, limits included and more preferably between 1.10 and 1.40 limits included.

**5.** The agglomerated zeolitic adsorbent according to one of claims 1 to 4, wherein said adsorbent comprises barium with a content of barium oxide (BaO) higher than 10%, preferably higher than 15%, more preferably higher than 20%, further preferably higher than 23%, even higher than 33% by weight relative to the total weight of the adsorbent, and advantageously the barium content is between 23% and 42%, typically between 30% and 40% by weight, limits included, relative to the total weight of the adsorbent.

**6.** The agglomerated zeolitic adsorbent according to one of claims 1 to 5, wherein said adsorbent comprises potassium with a content of potassium oxide $K_2O$ lower than 25%, preferably between 0 and 20%, more preferably between 0 and 15% by weight limits included, relative to the total weight of the adsorbent.

**7.** The agglomerated zeolitic adsorbent according to any of the preceding claims, wherein no zeolitic structure other than the faujasite structure, preferably no zeolitic structure other than the faujasite X structure is detected by X-ray diffraction.

**8.** The adsorbent according to any of the preceding claims wherein the weight fraction of FAU zeolite, the FAU zeolite being zeolite X, is higher than or equal to 80% relative to the total weight of the adsorbent.

**9.** Use of an adsorbent according to any of claims 1 to 8, in processes:

- to separate C8 aromatic isomer fractions, and xylenes in particular;
- to separate isomers of substituted toluene such as nitrotoluene, diethyltoluene, toluenediamine, and others;
- to separate cresols;
- to separate polyhydric alcohols.

**10.** The use according to claim 9, for the separation of para-xylene from aromatic isomer fractions having 8 carbon atoms.

11. Process for separating para-xylene from aromatic isomer fractions having 8 carbon atoms, using as para-xylene adsorption agent an agglomerated zeolitic adsorbent according to one of claims 1 to 8, in liquid phase or in gas phase.

12. The process for separating para-xylene from isomer fractions of aromatic hydrocarbons having 8 carbon atoms according to claim 11, in liquid phase by adsorption of para-xylene in the presence of a desorbent preferably selected from among toluene and n para-diethylbenzene.

13. The process according to any of claim 11 or 12 of simulated moving bed type, preferably of simulated counter-current type.

RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 3558730 A
- US 3558732 A
- US 3626020 A
- US 3663638 A
- US 3960774 A
- US 3878127 A
- US 2985589 A
- WO 2008009845 A
- WO 2014090771 A
- WO 2018002174 A

- US 20090326308 A
- DE 102018109701 A1
- US 7208651 B
- US 6136198 A
- CN 1191118 C
- US 7812208 B2
- US 2009326308 A
- US 2007224113 A
- WO 2009081022 A

**Littérature non-brevet citée dans la description**

- **GOMES et al.** *Adsorption,* 2006, vol. 12, 375

- **D. W. BRECK.** Zeolite Molecular Sieves. John Wiley and Sons, 1973, 314-315